# EUROPEAN PATENT APPLICATION

(11) **EP 3 653 045 A1**
(43) Date of publication of application: **20.05.2020**
(21) Application number: 18831567.5
(22) Date of filing: 09.07.2018
(51) Int. Cl.: A01G 33/00, A01H 13/00

(54) **PRODUCTION OF MACROALGAE CHIMERA**

(30) Priority: 13.07.2017 CL 20171827
(71) Applicant: Pontificia Universidad Católica De Chile, 8331150 Santiago (CL); Universidad De Chile, Santiago 8330015 (CL); Universidad Católica Del Norte, Antofagasta 1270709 (CL)
(72) Inventor: SANTELICES GONZÁLEZ, Bernabé, Santiago 8331150 (CL); GONZALEZ VÁSQUEZ, Alejandra, Santiago 8330015 (CL); VÁSQUEZ CASTRO, Julio, Antofagasta 1270709 (CL); TALA GONZÁLEZ, Fadia, Antofagasta 1270709 (CL)
(74) Representative: Herrero & Asociados, S.L.
(86) International application number: PCT/CL2018/050053
(87) International publication number: WO 2019/010588

(57) **Abstract**

The invention aims at a method for cultivating chimeric macroalgae, especially brown algae, where chimeric individuals are obtained in the laboratory and subsequently transferred to their definitive habitat. Chimeric macroalgae have advantages in their growth rate and greater resistance to environmental extremes in comparison to non-chimeric algae, so the method of the invention is especially useful for the commercial cultivation of these algae or for the recolonization of areas where the natural population of these algae has decreased significantly.

## Description

### FIELD OF THE INVENTION

The invention aims at a method for cultivating chimeric macroalgae, especially brown algae, where chimeric individuals are obtained in the laboratory and subsequently transferred to their definitive habitat. Chimeric macroalgae have advantages in their growth rate and greater resistance to environmental extremes in comparison to non-chimeric algae, so the method of the invention is especially useful for the commercial cultivation of these algae or for the recolonization of areas where the natural population of these algae has decreased significantly.

### BACKGROUND OF THE INVENTION

Chimeras consist of organisms that have more than one genotype and are produced by the somatic fusion or coalescence of two or more genetically different individuals of the same species, forming a single organism that possesses genetic heterogeneity. This phenomenon can occur in a specific group of organisms, including fungi, mold, grafted plants, colonial invertebrates (sponges, coral, etc.), and macroalgae.

Brown macroalgae (*"huiro"* kelp) are bioengineering organisms of coastal ecosystems, which dominate exposed rocky intertidal and subtidal environments, and provide numerous ecosystem services such as O₂ production, organic matter, substrate stabilization, habitat, shelter, spawning and larval settlement areas, and food for numerous fish and invertebrates (Rodríguez et. al. 2014; Vásquez et al. 2014). In Chile, the brown macroalgae of the order Laminariales (*Lessonia- "huiro negro*" kelp, "*huiro palo*" kelp; *Macrocystis- "huiro flotante"* kelp) and Fucales (*Durvillaea- "cochayuyo"* bull-kelp) represent the raw material for the alginate industry and biofuels, and are used as human food as well as in the cultivation of marine herbivores such as sea urchins and abalones (Buschmann et al. 2014). The brown algae fishery is focused in the north of the country, especially in the Atacama and Coquimbo regions, which represent the benthic fishery of most ecological, social, and economic importance. The mentioned regions contribute to 70% of the landing nationwide, reaching 400,000 tons/year.

Most of the commercialization of these brown macroalgae is for the extraction of alginate, which has a broad industrial platform monopolizing 60% of the global marine phycocolloid market. It is used in bakery and pastry for the preparation of stable fillings and restructured fruits. In dairy products, alginates are widely used in ice cream and low-fat spreads. For the year 2019, the market of alginates and their derivatives is expected to reach a value of USD 409.2 million, with a compound annual growth rate, or CAGR, of 3.8% in the 2014 - 2019 period. This market is dominated by North America and Europe, although developing countries emerge as exporters to these regions. The Asia-Pacific region is one of the most prominent producers and exporters, with projected growth for this market at a CAGR of 5.6% between 2014 and 2019. Globally, the market for alginates and their derivatives is driven by the demand for sodium alginate, used in textile, paper, ceramic, cosmetic, pharmaceutical and, of course, food and beverage applications. Other products that comprise this market are calcium, potassium, magnesium, zinc, and ammonium alginate, in addition to alginic acid, triethanolamine, and other alginates of high added value. In this context, Chile provides 10% of the world's biomass for alginate extraction, all from the exploitation of natural populations of the *"Huiro"* kelp (Vásquez et al. 2014).

Based on the above, natural populations are under enormous harvest pressure, causing the resource to be overexploited, affecting natural populations and its fishery. Consequently, there is a reduction in population size, genetic flow, and genetic diversity, which are exposed to inbreeding depression, low vigor, and adaptive capacity, variables that would lead to their extinction. Ecologically, ecosystem services diminish. Socioeconomically, fishermen extract less biomass, thus reducing economic income and quality of life, while the processing industry presents instability by reducing quantity, quality, and variety of raw material to extract bio-compounds. The State of Chile has generated sustainability strategies in management areas with biological and extractive closures, encouraging its potential cultivation for restocking. These sustainability strategies have focused on: 1) Management, protection, and conservation strategies of populations, with bio-ecological support, for their natural recovery, and 2) Pilot and mass cultivation of species with economic importance. Cultivation and restocking attempts have been carried out mainly in *Macrocystis pyrifera* with in-line (free-floating) crops, or seeding of spores in bare rock using monogenetic or low genetic variability plants (Vásquez & Tala 1995; Correa et al. 2006; Avila et al. 2010 FIC-R Tarapacá "Programa de Manejo, Cultivo y Repoblamiento para las Algas Pardas en la Region de Tarapacá; Westermeier et al. 2013 a, b). However, the cultivation in *Lessonia* species (*L*. *berteroana and L. spicata*) is little known and unsuccessful, has considered only one species (*L*. *berteroana*) applying suspended crops, seeding spores or juveniles. There are also no data on genetic and chemical diversity of crop seedlings, ignoring their impact on rocky ecosystems, so knowledge in *Lessonia* species for generating the cultivation and/or restocking is more limited despite its greater abundance and contribution in national landings (Hoffmann & Santelices 1982; Hoffmann et al. 1984; Avila et al. 1985; Edding et al. 1990; Vásquez & Tala 1995; Correa et al. 2006; Westermeier et al. 2006; Murúa et al. 2013). In this sense, Correa et al. (2006) propose as a method to restock, in areas impacted by mining activity on the coast of Chañaral (Atacama Region), the installation of individuals of *L. berteroana* inserted in a net by means of bolts to the rocky substrate. In these cases, the algae were able to re-adhere to the offered substrate and generate new holdfast tissue. However, the cultivation and restocking of exposed coastal rocky environments, subject to intense and frequent harvests in Lessonia species (*L*. *berteroana* and *L*. *spicata*), present numerous critical points, which so far have not improved the natural reserves, maintaining the species in an over-exploited resource status, a fact that has generated instability in the processing companies in terms of quantity and quality of the raw material produced.

Recently, it has been described that, in Chile, the juveniles and adults of Lessonia (*L. berteroana* and *L. spicata*) present natural processes of fusion of their fixation structures or holdfast coalescence (Vásquez 2008; Gonzalez et. al. 2014). These fusions generate genetically heterogeneous entities ("individuals") (Gonzalez et. al. 2014; Segovia et al. 2015) or chimeras (Gonzalez et. al. 2015a, b), which are frequent in natural populations (33-87%; Araujo & Faugeron 2016; Gonzalez & Santelices 2017). In these cases, chimerism occurs only with spatially close seedlings and, therefore, that belong to the same population. Within these, the holdfast is the area with chimerism having the greatest genetic diversity compared to the blades that are more genetically homogeneous (Gonzalez & Santelices 2017). These findings suggest that, at the economic and social level, most of the *Lessonia* species harvested and marketed at the national level would have chimerism, being the genetic composition of the seedlings that compose them and the implications on their chemical characteristics and production processes unknown. While at the ecological level, the chimeric holdfasts would represent a source of natural genetic diversity *in situ* with great importance in the resilience of the community and the ability of the seedlings to survive environmental changes.

In view of the above, the inventors presented the challenge of producing chimeras of these macroalgae *in vitro,* thus generating seedlings with a combinatorial character resulting from the somatic fusion of seedlings selected for specific characteristics such as: high growth rate, greater genetic diversity, production of chemical compounds, among others. This process of selecting strains confirming the chimeras does not occur in nature, since the fusion, when it occurs, is the result of a random process, mostly between spatially close organisms, without a specific selection of entities. Consequently, obtaining chimeras with the method of the invention would avoid the relentless pursuit of a natural "super strain" having at the same time all the specific characteristics desired by growers and the processing industries. In addition, obtaining a "super strain" by other methods can be a very slow and unsuccessful process, dependent on unknown genetic processes or requiring additional legal studies such as the safety of strains produced from hybrids or transgenically. Thus, obtaining chimeras by the method of the invention is a solution to the growing needs of manipulating and controlling the strains that are to be cultivated. In this sense, the inventors have found that with the method of the invention coalescence and chimera formation correlate positively and significantly with: survival, growth, and tolerance to environmental stress. Thus, the (multi-genetic) chimeric seedlings produced have better survival, faster growth, and more tolerance to environmental stress than single, unbound (genetically homogeneous) individuals.

Based on the above, the formation of chimeric seedlings with the method of the invention is an alternative that allows to improve the cultivation of Lessonia species (*L. berteroana* and *L. spicata*), as well as the restocking and restoration of coastal rocky ecosystems.

In this context, the production and restocking in a standardized way with chimeric seedlings allow a rapid production of biomass to be used in industrial processes, whose main target market may be the functional ingredients associated with causing beneficial effects on health (for example, alginates, antioxidants, pigments). On the other hand, considering that the restoking can be carried out through the use of chimeric plants, of high genetic and chemical diversity, the activity will not only contribute to the restoration of diversity and population density, but will also be of fast growth with greater tolerance to extreme factors (for example, herbivory, drying, increases in temperature, among others).

### DESCRIPTION OF THE FIGURES

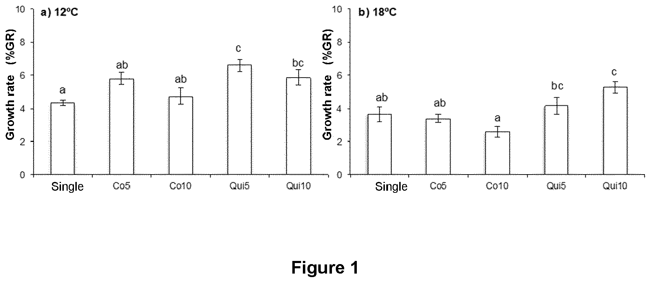

Figure 1. Growth rate under controlled laboratory conditions of *Lessonia spicata* sporophytes (20µmol photons m⁻² S⁻¹, 12L: 12°, 35‰, PSA culture medium). Single versus coalescent seedlings formed by 5 (Co5) and 10 (Co10) sporophytes and chimeric seedlings formed by 5 (Qui5) and 10 (Qui10) sporophytes are compared. a) Culture at 12°C. b) Culture at 18°C. Different letters indicate significant differences with P<0.05. At the two temperatures evaluated, chimeric algae have greater growth than single algae.

### DETAILED DESCRIPTION OF THE INVENTION

In order to obtain new chimeric organisms of high genetic diversity that will allow to solve the prior art problem of suitable algae cultivation, the inventors developed the method of the present invention. Where, in a first step, spores are obtained from different individuals of the same species, especially in brown algae of the group of *"huiro" kelp* (*Lessonia* and *Macrocystis* genera) and the group of *"cochayuyo"* bull-kelp (*Durvillaea antarctica*) Subsequently, these zygotes are cultivated in narrow proximity, in groups of up to 20 individuals, obtaining one or more chimeric organisms. As anticipated, conveniently and if possible, spores are obtained from individuals having desirable characteristics, such as high growth rate, high genetic diversity, or production of desirable chemical compounds, among others.

The method of the invention allows the generation of chimeric organisms with different characteristics to those of each of the parental plants, which result from the fusion, for example, of individuals with: 1) different ploidy levels (haploids versus diploids), 2) individuals with different growth rates, 3) different ages or sizes, 4) different initial number of individuals, 5) different genetic kinship between them, and 6) different populations of origin of the individuals involved. In this way, it is possible to modify the biological characteristics and, consequently, the economic characteristics of the resulting chimeric seedlings, opening the possibility of introducing algae cultivation production technology. The chimeras obtained can be selected, for example, by discarding individuals in poor physiological state. The chimeric seedlings are grown under controlled conditions and once they reach a size of 5 - 15 cm in length, they are moved to their final habitat, which can be an algae cultivation hatchery or their natural habitat, in the latter case, the seedlings are fixed to rocks at low intertidal and subtidal levels for their growth.

In this way, the invention discloses a process that allows the construction of selected chimeric *"huiro"* kelp plants, their cultivation, and then the transplanting for their growth to environments where these resources have been reduced, either by climatic events (for example, El Niño phenomenon) or by anthropogenic events (for example, overexploitation, contamination) allowing the recolonization of the environment but with selected seedlings. Alternatively, these plants can be grown in hatcheries specially adapted for this purpose, this option is highly convenient if the objective is the exploitation of juvenile plants that present specific metabolites or for obtaining a biomass with juvenile characteristics, for the pharmaceutical or food industry.

The chimeric macroalgae culture method of the invention comprises the following steps:
a. Obtaining haploid macroalgae spores from 2 to 10 individuals of the same species. Especially, the individuals are brown algae selected among species of the *Lessonia* and *Macrocystis* genera, or of the *Durvillaea antarctica* species.
b. Combining the spores obtained in the previous step in a container and keep them in the dark to allow the formation of new algae. This step is carried out in seawater or in liquid culture medium for algae, preferably the spores are kept in darkness for 12 to 36 hours, and subsequently in cycles of 12 hours of light/12 hours of darkness for 7 to 10 days.
c. Selecting the chimeric algae generated. This selection can be made by visual observation, for example, under a microscope, choosing only those chimeras that are well fused (without scar or evidence of contact area) and pigmented (without tissue bleaching) that is a symptom of good physiological condition of the chimera, in other words, individuals that are not fused or present depigmentation or bleaching of the holdfasts or blades are discarded;
d. Cultivating chimeric algae under controlled conditions. For example, the selected chimeric algae are incubated in 200 ml beakers, under controlled conditions of light intensity (20 ± 10 µmol photons m⁻² s⁻¹), constant photoperiod (12L:120), controlled temperature (12°C), with weekly replacement of culture medium (PSA) and a salinity of 35%;
e. Transferring the chimeric algae to their final habitat.

Conveniently, to transfer the chimeric algae to their definitive habitat, they are previously fixed to a support, which is chosen between plastic or metal grids, pieces of cloth, ropes or nets, for example. Chimeric plants can also be attached one by one to the rocks or simply be grown in an algae growth hatchery, so the use of a support is not always necessary.

Preferably, a plastic grid is used as support. In this case the seedlings are manually inserted into small pieces of plastic grid (for example, with a 5 mm opening), so that the holdfasts are firmly retained in the grid opening, preferably, groups of several individuals are inserted on each grid.

If the definitive habitat is the ocean, in intertidal environment, the supports with the seedlings are fixed to the rocks by chemical means, such as glues or putties, or by physical means such as staples, or by any other means available in the art, taking care that they are not toxic to algae or the environment.

The invention can be better understood according to the examples detailed below, which are illustrative and in no case limit the scope of the invention.

### EXAMPLES

The procedures for the production of chimeric seedlings and their transplantation to a definitive habitat comprise several steps, namely:
1. Production of chimeric seedlings and their *in vitro* culture
   1.1. Collection of biological material from the target species
   1.2. Haploid spore release
   1.3. Formation of microscopic gametophytes
   1.4. Isolation of single, coalescing, and chimeric sporophytes
   1.5. Cultivation of select sporophytes *in vitro*
2. Transfer and transplantation of chimeric seedlings to the intertidal and subtidal rocky areas.
   2.1. Seedling adhesion using plastic grids
   2.2. Seedling adhesion by manual fixation

### 1. Production of chimeric seedlings and their in vitro culture

### 1.1 Collection of biological material from the target species

The species used correspond to the *Lessonia nigrescens* species ("*huiro negro"* kelp, *"huiro chascón"* kelp) conformed by of *L. berteroana,* whose geographical distribution range ranges from 30°S to the extreme north of Chile, and *L. spicata*, which extends from 30°S to the South (Tellier et al. 2009, González et al. 2012). The genus *Lessonia Bory*, is native to the South Pacific and belongs to the order Laminariales.

Once the location(s) is selected, the reproductive material (fronds or blades with sori) was collected from different plants. A holdfast with several blades (>5) is considered as a discrete sampling unit. 10 - 20 holdfasts separated by a minimum distance of 2 m between each plant were randomly selected to increase the probability of selection of genetically different individuals. Only one reproductive frond was removed from each selected holdfast.

### 1.2 Haploid spore release

### (Avila et al. 1985, Martinez and Santelices 1998, Avila et al. 2010, Oppliger et al. 2011, Gonzalez et al. 2014)

Once the reproductive material was collected, the frond and the sorus were washed *in situ* three times with running water (sterilized) and brushed with a brush. The washed tissue was then placed in plastic trays in the sun for dehydration for approximately 1 - 2 hrs. When the tissue dried, reproductive tissue was extracted with a punch (1 -1.5 cm diameter) that was placed in Falcon tubes (BD Biosciences, San Jose, CA, USA), with sterile seawater, at low temperature (5 - 8°C) and with a slide inside. The rehydration and thermal shock generate the release of haploid spores (female and male gametes).

For comparative effects, single seedlings, coalescent seedlings but of low genetic diversity (formed by spores of the same plant), and chimeric seedlings (formed by spores of several plants, with greater genetic diversity) were obtained. For single and coalescing plants, only 1 - 2 pieces of reproductive tissue from the same seedling were placed in the Falcon tube. For the generation of chimeric seedlings, 5 - 10 pieces of reproductive tissue from different and distant plants, placed at a minimum of 2 m between them, were placed in the Falcon tube.

All tubes were transported in thermal boxes (5 - 8°C) and darkness to the laboratory (2 - 3 hrs). There, the pieces of tissue were removed from the tubes and were kept in growth chambers in the dark for 18 to 24 hours.

### 1.3 Formation of microscopic gametophytes

### (Avila et al. 1985, Avila et al. 2010, Oppliger et al. 2011, Gonzalez et al. 2014)

After 18 - 24 hours of incubation in the dark, all Falcon tubes were changed to seawater, replacing it with the artificial culture medium PSA (Provasoli 1968). These tubes were then horizontally incubated in motion, using a 100 rpm shaker (SO1 orbital shaker, Stuart Scientific, Stone, Staffordshire, UK), under controlled conditions (12° ± 2°C, irradiance of 20 ± 10 µmol photons m⁻² s⁻¹, photoperiod 12L: 12D), until the development of gametophytes (microscopic, haploid phase). The culture medium (PSA) was changed twice a week until the appearance of seedlings or sporophytes, which under these conditions appeared in 7 - 10 days.

### 1.4 Isolation of single, coalescing, and chimeric sporophytes

### (Gonzalez et al. 2014)

Once the sporophytes appeared (blade length of approx. 0.2 - 0.5 cm), they were isolated according to the genetic nature of interest. They were of three types: single (control), coalescent or chimeric. For this, on each slide, only the sporophyte(s) of interest is/are left adhered to the substrate and will continue to be evaluated during its development. This includes:
a) Single (monogenetic) seedlings: obtained by isolating independent sporophytes from a single plant.
b) Coalescent (multi-genetic, with high kinship) seedlings: obtained by isolating fused sporophytes from a single plant.
c) Chimeric seedlings (multi-genetic, with low kinship): obtained by isolating fused sporophytes from 5-10 different plants.

Slides with isolated sporophytes were placed in 200 ml beakers with PSA culture medium.

### 1.5 Cultivation of select sporophytes

The sporophytes selected for future growth were incubated in 200 ml beakers, under controlled conditions of light intensity (20 ± 10 µmol photons m⁻² s⁻¹), constant photoperiod (12L:12O), controlled temperature (12°C), with weekly replacement of culture medium (PSA) and a salinity of 35%.

The experimental results of this laboratory culture (see Fig. 1) indicate that:
a) Single seedlings grow at a slower rate than coalescent (monogenetic) seedlings and chimeric seedlings (Fig. 1a).
b) There was no significant effect on the number of individuals that formed a coalescent (5 or 10) or a chimeric seedling (5 or 10, Fig. 1a)
c) Growth rates are reduced when incubation is performed at 18°C. In general, temperatures above 14°C are stressful for *"huiro"* kelp species (Fig. 1b).
d) The chimeric seedlings show the highest growth rates. Comparisons of these with the growth of single seedlings, always showed to be significantly higher. The differences with chimeric seedlings can become statistically significant depending on the number of sporophytes or individuals that conform the seedling (5 or 10) and the temperature at which the crop is grown.
e) Under stressful temperatures (18°C), chimeric seedlings grew more than single and coalescing seedlings (Fig. 1b).

Consequently, the use of chimeric seedlings for growing and restocking would have a higher growth rate and genetic variation, reducing inbreeding depression and loss of vigor of the strains to be cultivated. Additionally, they show greater resistance to the increase in temperature than single seedlings, a fact that seems to be a recurring process on the Chilean coasts in the face of continuous environmental changes due to climate change.

### 2. Transfer and transplantation of chimeric seedlings to the intertidal and subtidal requirements.

### 2.1 Seedling adhesion using plastic grids

The seedlings were inserted in groups of 9 individuals in small squares of 3.2 x 3.2 cm of plastic grid of 5 mm of opening. Each plant was inserted by hand into the grid, so that the holdfasts are firmly retained in the grid opening. These groups of plants inserted in grids were transported to the installation sites, in closed plastic containers at a temperature between 10 and 15°C. In rocky intertidal areas of the exposed coast, these grids with seedlings of *Lessonia* species, were adhered to the substrate with Sherwin Williams Underwater Epoxy Putty.

The vertical growth of the holdfast allows the adhesion of the seedling to the rocky substrate, while the horizontal growth allows the fusion of holdfasts of adjacent plants. Between 30 and 45 days from the installation of the grids with seedlings, the fusion of all the seedlings takes place, forming a multigenetic unit or chimeric unit. These chimeric units showed a survival of 80% in the next 6 months of control, at which time the single holdfast of the chimeric units had overgrown the grid and the putty, becoming indistinguishable from plants of natural populations.

### 2.2 Seed adhesion by manual fixation

The seedlings were fixed manually, one by one, on the slightly damp surface of the rock, in exposed intertidal environments. *Lessonia* seedlings were fixed with an organic cyanoacrylate glue (Seachem ReefGlue™). The glue is applied on the holdfasts of the plants, which are manually and individually glued the substrate. Each plant with cyanoacrylate glue is pressed and held on the substrate for 30 - 40 seconds to allow the seedling to adhere to the rock. Groups of 5 seedlings, attached to the rock at distances no greater than 1 cm, form planting units. A variable number between 3 and 5 planting units were installed in the rocky substrate of the exposed intertidal, 10 cm away from each other. Inside each planting unit, the seedling holdfasts merged within the next 20 days from installation. Fusion between planting units occurs between 45 and 50 days after installation. These partial fusions (inside the planting units) and total (between planting units), form multigenetic chimeric units.

### Recommendations for adhesion transplantation processes

The installation of juvenile sporophytes should be carried out during the lowest tides of the month, and in the lowest possible portion of the intertidal zone. In order to increase the adhesion and survival of the adhered seedlings, it is preferable that the sporophytes be installed in cracks or irregularities of the substrate. Before the transplant, it is convenient that in the chosen intertidal rocky area, a manual exclusion of benthic herbivorous invertebrates is performed, at the beginning and every time the tide permits.

### References cited

- Araujo Casares F, Faugueron S. 2016. Higher reproductive success for chimeras than solitary individuals in the kelp Lessonia spicata but no benefit for individual genotypes. Evol Ecol. 2016; DOI 10.1007/s10682-016-9849-0.
- Avila M, Merino C, Guissen K & Piel MI. 2010. Manual De Cultivo De Macroalgas Pardas: Desde EI Laboratorio AI Océano. Universidad Arturo Prat. Intellectual Property Registration No. 212093, Chile.
- Avila M, Hoffmann AJ & Santelices B. 1985. Interacciones de temperatura, densidad de flujo fotónico y fotoperiodo sobre el desarrollo de etapas microscópicas de Lessonia nigrescens (Phaeophyta, Laminariales). Rev. Chil. Hist. Nat. 58:71-82.
- Buschmann AH, Prescott S, Potin P, Faugeron S, Vasquez JA, Camus C, Infante J, Hernandez-Gonzalez M, Gutierrez A, & Varela DA. 2014. The status of kelp exploitation and marine agronomy, with emphasis on Macrocystis pyrifera in Chile. Adv. Bot. Res. 71: 161-188.
- Correa JA, Lagos NA, Medina MH, JC Castilla, M Cerda, M Ramírez, E Martinez, S Faugeron, S Andrade, R Pinto & Contreras L. 2006. Experimental transplants of the large kelp Lessonia nigrescens (Phaeophyceae) in high-energy wave exposed rocky intertidal habitats of northern Chile: Experimental, restoration and management applications. J. Exp. Mar. Biol. Ecol. 335: 13-18.
- Edding M, Venegas M, Orrego P & Fonck E. 1990. Culture and growth of Lessonia trabeculata juvenile sporophytes in northern Chile. Hydrobiologia 204/205,361:366.
- Gonzalez AV, Beltrán J, Hiriart-Bertrand L, Flores V, de Reviers B, Correa JA & Santelices B. 2012. Identification of cryptic species in the Lessonia nigrescens complex (Phaeophyceae, Laminariales). J. Phycol. 48:1153-1165.
- Gonzalez AV, Borras-Chávez R, Beltrán J, Flores V, Vásquez JA & Santelices B. 2014. Morphological, ultrastructural, and genetic characterization of coalescence in the intertidal and shallow subtidal kelps Lessonia spicata and L. berteroana (Laminariales, Heterokonthophyta). J. Appl. Phycol. 26: 1107-1113.
- Gonzalez AV, Beltrán J, Flores V & Santelices B. 2015a. Morphological convergence in the inter-holdfast coalescence process among kelp and kelp-like seaweeds (Lessonia, Macrocystis, Durvillaea). Phycologia 54 (3): 283-291.
- Gonzalez AV, Flores V, Beltrán J. & Santelices B. 2015b. Discos quiméricos de macroalgas pardas como reservorios de diversidad genética y su importancia en conservación. XXII Annual Meeting of The Ecological Society of Chile. Olmué, November 03 - 06. Chile.
- Gonzalez AV & Santelices B. 2017. Frequency of chimerism in populations of the kelp Lessonia spicata in central Chile. PLoS ONE 12(2): e0169182. doi:10.1371/journal. pone. 0169182.
- Hoffmann AJ & Santelices B. 1982. Effects on light intensity and nutrients on gametophytes and gametogenesis of Lessonia nigrescens Bory (Phaeophyta). J. Exp. Mar. Bio. Ecol. 60:77-89.
- Hoffmann AJ, M Avila & Santelices B. 1984. Interactions of nitrate and phosphate on the development of microscopic stages of Lessonia nigrescens Bory (Phaeophyta). J. Exp. Mar. Biol. Ecol. 78: 177-186.
- Hurd CL, Harrison PJ, Bischop K, Lobban CS (2014) Seaweed ecology and physiology, 2nd edn. Cambridge Univ. Press, Cambridge, UK.
- Martinez, E.A. and B. Santelices. 1998. Selective mortality on haploid and diploid microscopic stages of Lessonia nigrescens Bory (Phaeophyta, Laminariales). J. Exp. Mar. Biol. Ecol. 229: 219-239.
- Provasoli L. 1968. Media and prospects for cultivation of marine algae. In: A. Watanabe and A. Hattori (eds). Cultures and collections of algae. The Japanese Society of Plant Physiologists, Tokyo. pp. 47-74.
- Rodriguez DC, Oróstica MH, & Vásquez JA. 2014. Coalescence in wild organisms of the intertidal population of Lessonia berteroana in northern Chile: management and sustainability effects. J. Appl. Phycol. 26: 1115-1122.
- Segovia NI, Vásquez JA, Faugeron S, Haye PA. 2015. On the advantage of sharing a holdfast: effects of density and occurrence of kin aggregation in the kelp Lessonia berteroana. Mar. Ecol. 36 (4): 1107-1117.
- Sokal RR, FJ Rohlf 1995 Biometry: the principles and practice of statistics in biological research. WH Freeman, New York.
- Tellier F, Meynard AP, Correa JA, Faugeron S, & Valero M. 2009. Phylogeographic analyses of the 30° S south-east Pacific biogeographic transition zone establish the occurrence of a sharp genetic discontinuity in the kelp Lessonia nigrescens: vicariance or parapatry. Mol. Phyl. Evol. 53: 679-93.
- Vásquez JA. 2008. Production, use and fate of Chilean brown seaweeds: resources for a sustainable fishery J. Appl. Phycol. 20: 457-467.
- Vásquez JA, Zuñiga S, Tala F, Piaget N, Rodriguez DC & Vega JMA. 2014. Economic evaluation of kelp forest in northern Chile: values of good and service of the ecosystem. J. Appl. Phycol. http://dx.doi.org/10.1007/s10811-013-0173-6.
- Vásquez JA & Tala F. 1995. Experimental repopulation of Lessonia nigrescens (Phaeophyta, Laminariales) in intertidal areas of northern Chile. J. Appl. Phycol. 7:347-349.
- Westermeier R, P Murúa, DJ Patiño, L Muñoz, A Ruiz, C Atero, & Müller DG. 2013a. Utilization of holdfast fragments for vegetative propagation of Macrocystis integrifolia in Atacama, Northern Chile. J. Appl. Phycol. 25 (2): 639-642.
- Westermeier R, P Murúa, DJ Patiño, L Muñoz, C Atero, & DG Müller. 2013b. Repopulation techniques for Macrocystis integrifolia (Phaeophyceae: Laminariales) in Atacama, Chile. J. Appl. Phycol. 26 (1): 511-518.
- Westermeier R, Patiño D, Piel M, Maier I, & Mueller D. 2006. A new approach to Kelp mariculture in Chile: production of free-floating sporophyte seedlings from gametophyte cultures of Lessonia trabeculata and Macrocystis pyrifera. Aquaculture Res. 37:164-171.
- Murúa P, R Westermeier, DJ Patiño, & Müller DG. 2013. Culture studies on early development of Lessonia trabeculata (Phaeophyceae, Laminariales): seasonality and acclimation to light and temperature. Phycological Res. 61 (2): 145-153.

## Claims

1. Method for cultivating chimeric macroalgae **CHARACTERIZED in that** it comprises:
a. Obtaining haploid macroalgae spores from 2 to 10 individuals of the same species,
b. Combining the spores obtained in the previous step in a container and keep in the dark to allow the formation of new algae,
c. Selecting the chimeric algae generated,
d. Cultivating chimeric algae under controlled conditions,
e. Transferring the chimeric algae to their final habitat.

2. Method according to claim 1 **CHARACTERIZED in that** in step a), the individuals are brown algae selected among species of the *Lessonia* and *Macrocystis* genera, or of *the Durvillaea antarctica* species.

3. Method according to claim 1 **CHARACTERIZED in that** step b) is carried out in seawater or in liquid culture medium for algae and kept in darkness for 12 to 36 hours, and subsequently in cycles of 12 hours of light/12 hours of darkness for 7 to 10 days.

4. Method according to claim 1 **CHARACTERIZED in that** in step d) the sporophytes selected for future growth are incubated in 200 ml beakers, under controlled conditions of light intensity (20 ± 10 µmol photons m⁻² s⁻¹), constant photoperiod (12L: 120), controlled temperature (12°C), with weekly replacement of culture medium (PSA), and a salinity of 35%.

5. Method according to claim 1 **CHARACTERIZED in that** in step c) the chimeric algae are selected by visual observation, discarding those with a fusion scar or with depigmentation or bleaching of holdfasts or blades.

6. Method according to claim 1 **CHARACTERIZED in that** in step e) the algae are fixed to a support.

7. Method according to claim 6 **CHARACTERIZED in that** the support are plastic or metal grids, pieces of cloth, ropes or nets.

8. Method according to claim 7 **CHARACTERIZED in that** the support is preferably a plastic grid.

9. Method according to claim 8 **CHARACTERIZED in that** the seedlings are manually inserted into the openings of small pieces of plastic grid so that the holdfasts are firmly retained in the grid opening.

10. Method according to claim 9 **CHARACTERIZED in that** several individuals are inserted per grid.

11. Method according to claim 6 **CHARACTERIZED in that** in step e) if the final habitat is the ocean, in intertidal environment, the supports with the seedlings are fixed to rocks by chemical means, such as glues or putties, or by physical means, such as staples, or others, taking care that they are not toxic to organisms or the environment.
